# EUROPEAN PATENT APPLICATION

(11) **EP 1 197 230 A1**
(43) Date of publication of application: **17.04.2002**
(21) Application number: 00950130.5
(22) Date of filing: 30.06.2000
(51) Int. Cl.: A61L 15/00, A61L 15/20, A61L 15/44

(54) **MEDICAL FABRIC WITH VIRUCIDAL AND ANTIMICROBIAL ACTION AND ARTICLES THEREFROM**

(30) Priority: 30.06.1999 RU 99113369
(71) Applicant: Zakrytoe Aktsionernoe Obschestvo Nauchno Proizvodstvennoe Meditsinskoe Predpriyatie "Bioekran", Moscow, 103051 (RU)
(72) Inventor: KOZINDA, Zinaida Julianovna, Moscow, 125080 (RU); SUVOROVA, Elena Grigorievna, Moscow, 105215 (RU); STRUKOV, Mikhail Vasilievich, Moskovskaya obl., 141070 (RU); SEDOV, Alexandr Vladimirovich, Moscow, 123481 (RU); GONCHAROV, Sergei Fedorovich, Moscow, 107014 (RU)
(74) Representative: Leach, John Nigel
(86) International application number: RU0000267
(87) International publication number: WO0100252

(57) **Abstract**

The invention relates to the field of medicine, namely, to prevention and prophylaxis of infection/inflammatory diseases caused by impact of adverse microbiological factors, by means of providing a medical material and products made therefrom having virucidal and antimicrobial activity.

The medical material on the basis of natural fabrics with an antimicrobial substance as the antimicrobial substance comprises a quadruple ammonium base having 10-18 carbon atoms, Catamin AB, and a polyatomic alcohol, with the following component ratio, wt. % of the material weight:
Quadruple ammonium base having 10-18 carbon atoms,

| | |
|---|---|
| Catamin AB | - 0.06-0.75 |
| Polyatomic alcohol | - 0.03-0.038 |

## Description

The present invention relates to the field of medicine, namely, to prevention and prophylaxis of infection/inflammatory diseases caused by impact of adverse microbiological factors, by means of providing a medical material and products made therefrom having virucidal and antimicrobial activity.

Clothes, personal hygiene articles such as bed-linen, towel, diapers, napkins, gloves, hosiery, etc., having antimicrobial activity are requisite not only in medical institutions, such as hospitals, burn centres, maternity hospitals, in emergency situations, in cases of epidemics, but also in household. In household, these articles are used as prophylactic means reducing the possibility of falling ill and relapse occurrences. For workers of medical institutions these article are requisite for diminishing the danger of spreading in-hospital infection, being in a prolonged position at the working place without the possibility to change the clothes, as articles from antimicrobial material are conducive to disruption of epidemical path of disease transfer through human clothes, as well as to prevent repeated autoinfection.

An antimicrobial material comprising a base of natural fibres and sanguiritrine which is used both in medicine and in sanitary. The method for producing this material, comprising impregnating the natural fibres with sanguiritrine and subsequent drying it. (USSR author's certificate 1771750, Cl. A 61 L 15/00, 30.10.1992).

However, these modifications do not possess antimicrobial activity to a vast group of microorganisms and fungi and fail to retain their properties after washing.

The known materials have antimicrobial activity with respect to gram-positive, gram-negative microorganisms and fungi, including viruses/infection agents not having cellular structure and being distinguished by extremely small sizes and capable to pass through bacteria retaining filters and pertaining to dressing.

The technical solution closest to the claimed one is the textile material having antimicrobial activity and made from bactericidal fibres of 3,4,4-trichluorocarbanilide, tin-organic compounds and hexachlorophene. This material is intended for clothes and underwear sewing. However, these materials are toxic which does not make it possible to employ them as a prophylactic antimicrobial material, and they have a narrow activity range. (USSR author's certificate 317660, Cl. A 61 L 15/00, 19.10.1971).

The object of the present invention is providing a material and personal hygiene articles made from it that would have biological activity with respect of a number of gram-positive, gram-negative groups, pathogenic fungi and with respect to group F/RK/8/34 microvirues, eteroviruses (type III polyomyelitis and A hepatitis) without any toxic effect on the human organism and laundry-resistant.

This objet is achieved by manufacturing the article (clothes, personal hygiene articles such as bed-linen, towel, diapers, napkins, gloves, hosiery, etc.) from a fabric containing a sorbed or fabric chemically associated biologically active agent, a quadruple ammonium base with 10-18 carbon atoms, Catamin AB and polyatomic alcohol with the component wt. % ratio of:

| | |
|---|---|
| Catamin AB | - 0.06-0.075 |
| Polyatomic alcohol | - 0.03-0.038 |

Catamin AB, alkyldimethylbenzyl ammonium chloride (synonym, Rockal) is an antiseptic and relates to cationic surfactants. [R(CH₃)₂(CH₂C₆H₅)N]⁺Cl⁻ R-mixture of C₁₀H₂₁-C₁₈H₃₇ or C₁₂H₂₅-C₁₄H₂₉ straight-chain alkyl groups. Medium molar weight of Catamin AB is 361±15 g/mole. Technical specification TU 2482-012-1316 4401-94.

Cotton, wool, polyamide, mixed fibre materials and other materials used in medicine may serve as the material.

### Embodiment 1 of the Invention

Cotton fabric is periodically treated with a 0.9% antiseptic Catamin AB aqueous solution of the solids weight in the presence of 0.038% of polyatomic alcohol. Sorption is carried out during a time period of more than 30 minutes. After which the material produced is dried. Whereby concentration of the sorbed active substance is about 0.06% of the material weight.

### Embodiment 1 of the Invention

Woollen fabric is treated according to operation of the method described in Embodiment 1, whereby antiseptic is used in concentration of 1.5% of solids weight, the polyatomic alcohol is in the amount of 0.038%. Concentration of the active sorbed substance on the material is 0.075% of the material weight.

### Embodiment 3 of the Invention

Polyamide fabric is treated with Catamin AB 1.2% and polyatomic alcohol 0.035% aqueous solution periodically for 60 minutes, followed by drying the material produced. Concentration of Catamin AB was 0.07% of the material weight.

Examples of producing and properties of the material are provided in the table.

The material produced is used to manufacture articles such as clothes, personal hygiene items, bed-linen, towel, diapers, napkins gloves, hosiery, etc.

This material has no toxic effect on humans, does not affect human immune system, promotes more rapid curing of the patient and prevents disease relapses, which is supported by its use in burn centres and by prophylactic use under household conditions.

Ten patients having II and III degree bums were being cured on cots provided with sheets made from this material. Observations have shown that epidermis recovery occurs after 10-12 days. Healing is without secondary stretching.

When using medicinal gloves and socks made from this material, after nail removal in treating nail mycosis, a significant relapse reduction (down to 75%) was observed as well as accelerated recovery of nail bed epidermis.

The use of antimicrobial clothes in clinics by medical personnel 2-4 times reduces seeding of smocks, suits and other clothes. Thus, it enhances protection of medical personnel against microbial factor and prophylaxis of in-hospital infections.

Thus, articles manufactured from this material are capable of speeding-up the process of curing patients having diseases caused by a number of gram-positive and gram-negative groups of microorganisms, pathogenic fungi, as well of acting prophylactically when there exists the possibility of their proliferation.

**Table**

| Nº | catamin content | Area of inhibition of organism growth, mm | | | | | |
|---|---|---|---|---|---|---|---|
| | | golden staphylococcus | | Colon bacillus | | Yeast fungi | |
| | % of material weight | before washing | after 15 washings | before washing | after 15 washings | before washing | after 15 washings |
| cotton | | | | | | | |
| 1 | 0,06 | 4 | 4 | 2,5 | 1,5 | 2,5 | 2 |
| 2 | 0,065 | 5,5 | 5 | 2,5 | 2 | 3 | 3 |
| 3 | 0,07 | 6,5 | 6 | 3,5 | 3 | 3,5 | 3 |
| 4 | 0,075 | 7 | 7 | 4,5 | 4 | 4 | 3,5 |

| wool | | | | | | | |
|---|---|---|---|---|---|---|---|
| 1 | 0,06 | 4 | 3,5 | 2,5 | 2 | 2,5 | 2,5 |
| 2 | 0,065 | 5 | 4,5 | 3 | 2,5 | 3 | 2,5 |
| 3 | 0,07 | 6,5 | 6 | 3,5 | 3 | 4 | 3,5 |
| 4 | 0,075 | 7,5 | 7 | 4 | 3,5 | 4 | 4 |

| polyamide | | | | | | | |
|---|---|---|---|---|---|---|---|
| 1 | 0,06 | 4 | 3,5 | 2,5 | 1,5 | 2,5 | 2 |
| 2 | 0,065 | 4 | 3 | 3 | 2,5 | 3 | 2,5 |
| 3 | 0,07 | 4,5 | 4 | 3,5 | 3 | 3,5 | 3 |
| 4 | 0,075 | 5 | 5 | 4,5 | 4 | 4 | 4 |

| cotton 50% - polyamide 50% | | | | | | | |
|---|---|---|---|---|---|---|---|
| 1 | 0,06 | 4,0 | 3,50 | 2,5 | 2,0 | 2,5 | 2,0 |
| 2 | 0,065 | 4,5 | 4,00 | 2,7 | 2,5 | 3,0 | 2,5 |
| 3 | 0,07 | 5,0 | 4,50 | 3,0 | 3,0 | 3,5 | 3,0 |
| 4 | 0,075 | 6,0 | 5,00 | 4,0 | 3,5 | 4,0 | 3,5 |

| cotton 50% - polyester 50% | | | | | | | |
|---|---|---|---|---|---|---|---|
| 1 | 0,06 | 3,0 | 3,0 | 2,5 | 1,5 | 2,0 | 1,5 |
| 2 | 0,065 | 3,6 | 3,5 | 2,5 | 2,0 | 2,0 | 2,0 |
| 3 | 0,07 | 4,0 | 3,5 | 3,0 | 2,0 | 2,5 | 2,0 |
| 4 | 0,075 | 4,2 | 4,0 | 3,0 | 2,5 | 3,5 | 2,5 |

| wool 50% - polyamide 50% | | | | | | | |
|---|---|---|---|---|---|---|---|
| 1 | 0,06 | 4,0 | 3,5 | 2,5 | 2,0 | 2,0 | 2,0 |
| 2 | 0,065 | 4,5 | 3,5 | 3,0 | 2,0 | 2,0 | 2,0 |
| 3 | 0,07 | 5,0 | 4,0 | 3,5 | 2,5 | 3,5 | 3,0 |
| 4 | 0,075 | 5,5 | 4,5 | 4,0 | 3,0 | 4,0 | 3,0 |

## Claims

1. A medical material on the base of natural fabrics with an antimicrobial substance, **characterised in that** it comprises, as the antimicrobial substance, a quadruple ammonium base having 10-18 carbon atoms, Catamin AB, and polyatomic alcohol with the following component ratio, wt. % of the material weight:
Quadruple ammonium base having 10-18 carbon atoms,
| | |
|---|---|
| Catamin AB | - 0.06-0.75 |
| Polyatomic alcohol | - 0.03-0.038 |

2. An article made from a medical material on the base of natural fabrics with an antimicrobial substance, **characterised in that** it comprises, as the antimicrobial substance, a quadruple ammonium base having 10-18 carbon atoms and polyatomic alcohol with the following component ratio, wt. % of the material weight:
Quadruple ammonium base having 10-18 carbon atoms,
| | |
|---|---|
| Catamin AB | - 0.06-0.75 |
| Polyatomic alcohol | - 0.03-0.038 |

3. An article according to claim 2, **characterised in that** it is formed as surgical articles such as surgical sheets, surgical clothes, mask, covers.

4. An article according to claim 2, **characterised in that** it is formed as private hygiene articles such as bed-linen, towel, diapers, gloves, hosiery.

5. An article according to claim 2, **characterised in that** it is formed as underwear.
